# DEMANDE DE BREVET EUROPEEN

(11) **EP 2 564 802 A1**
(43) Date de publication de la demande: **06.03.2013**
(21) Numéro de dépôt: 12180316.7
(22) Date de dépôt: 13.08.2012
(51) Int. Cl.: A61B 19/00, A61B 17/15, A61B 17/17

(54) **Procédé de fabrication d'un guide de positionnement personnalisé**

(30) Priorité: 29.08.2011 FR 1102620
(71) Demandeur: I.M.A.G.E., 07500 Guilherand-Granges (FR)
(72) Inventeur: Cladiere, Franck, 07500 GUILHERAND GRANGES (FR); Biette, Grégory, 64990 VILLEFRANQUE (FR); Copieau, Patrick, 37230 LUYNES (FR); Martres, Sébastien, 83400 Hyères (FR); Elhage, Richard, 59130 LAMBERSART (FR); Verdot, François Xavier, 42000 SAINT ETIENNE (FR); Fournol, Stéphane, 77150 Lesigny (FR); Bussiere, Christophe, 71640 DRACY LE FORT (FR); Roussignol, Xavier, 76250 DEVILLE LES ROUEN (FR)
(74) Mandataire: Parzy, Benjamin Alain

(57) **Abrégé**

L'invention concerne un procédé de fabrication d'un guide de positionnement (10) personnalisé d'au moins un organe de positionnement sur une extrémité distale (1) d'un fémur ou sur une extrémité proximale d'un tibia d'un patient, le procédé comportant l'étape (200) de modéliser l'extrémité souhaitée en un modèle virtuel en trois dimensions à partir d'au moins une image médicale de ladite extrémité. Selon l'invention, le procédé comporte les étapes de :
- rapporter (300) sur le modèle virtuel de l'extrémité un guide de positionnement virtuel prédéterminé en trois dimensions ;
- enlever (400) virtuellement du guide de positionnement virtuel prédéterminé une portion intersectée par le modèle virtuel de l'extrémité pour obtenir un guide de positionnement virtuel personnalisé;
- fabriquer (500) le guide de positionnement personnalisé par frittage de poudre à partir du guide de positionnement virtuel personnalisé.

## Description

L'invention concerne un procédé de fabrication d'un guide de positionnement personnalisé.

Dans la présente demande, on se référera au système de référence anatomique classique dans lequel le patient est debout face à un observateur. Dans ce système de référence, il est rappelé que l'extrémité distale d'un fémur désigne l'extrémité du fémur associée au genou et que l'extrémité proximale d'un tibia désigne l'extrémité du tibia associée au genou.

### ARRIERE PLAN TECHNOLOGIQUE DE L'INVENTION

Une pose d'une prothèse totale de genou vise à restaurer une articulation entre un fémur et un tibia d'un patient.

Afin de pouvoir poser la prothèse totale, un chirurgien doit préparer l'extrémité distale du fémur et l'extrémité proximale du tibia à recevoir respectivement la partie fémorale et la partie tibiale de la prothèse totale. A cet effet, le chirurgien réalise différentes coupes sur les deux extrémités citées.

Or pour pouvoir être portée sans douleur ni gêne par le patient, une prothèse de genou doit reproduire le plus précisément possible le genou qu'elle remplace. Il est donc primordial de réaliser très précisément les différentes coupes afin de positionner au mieux la prothèse totale de genou.

Pour aider le chirurgien à réaliser les différentes coupes, il existe des guides de coupe permettant de guider le geste du chirurgien. Les guides de coupe sont disposés temporairement sur les extrémités à préparer grâce à des organes de positionnement, telle que des broches, préalablement implantés sur lesdites extrémités. A cet effet, il existe des guides de positionnement de broches qui aident le chirurgien à forer les extrémités pour implanter les broches. Ces guides de positionnement sont adaptés au patient afin de permettre un positionnement des broches propre au patient de sorte que les coupes réalisées soient les plus adaptées possible à l'anatomie du patient.

### OBJET DE L'INVENTION

Un but de l'invention est de proposer un procédé de fabrication d'un guide de positionnement personnalisé d'au moins un organe de positionnement qui soit simple et rapide à mettre en oeuvre.

### BREVE DESCRIPTION DE L'INVENTION

En vu de la réalisation de ce but, on propose un procédé de réalisation d'un guide de positionnement personnalisé d'au moins un organe de positionnement sur une extrémité distale d'un fémur ou sur une extrémité proximale d'un tibia d'un patient, le procédé comportant l'étape de modéliser l'extrémité souhaitée en un modèle virtuel en trois dimensions à partir d'au moins une image médicale de ladite extrémité. Selon l'invention, le procédé comporte les étapes de :
- rapporter sur le modèle virtuel de l'extrémité un guide de positionnement virtuel prédéterminé en trois dimensions ;
- enlever virtuellement du guide de positionnement virtuel prédéterminé une portion intersectée par le modèle virtuel de l'extrémité pour obtenir un guide de positionnement virtuel personnalisé;
- fabriquer le guide de positionnement personnalisé par frittage de poudre à partir du guide de positionnement virtuel personnalisé.

Les inventeurs ont pu constater qu'il était bien plus aisé de réaliser un guide de positionnement adapté à l'anatomie d'un patient grâce au procédé de l'invention que par exemple, déterminer un guide de positionnement virtuel personnalisé uniquement à partir de la surface externe du modèle virtuel de l'extrémité.

### BREVE DESCRIPTION DES DESSINS

L'invention sera mieux comprise à la lumière de la description qui suit d'une mise en oeuvre particulière non limitative de l'invention en référence aux figures ci-jointes parmi lesquelles :
- la figure 1 est une vue en trois dimensions schématisée d'une extrémité distale d'un fémur sur laquelle est disposé un guide de positionnement personnalisé de broches fabriqué selon le procédé de l'invention, quatre broches étant prêtes à être positionnées sur ladite extrémité ;
- la figure 2 est un schéma illustrant les différentes étapes de fabrication du guide de positionnement personnalisé illustré à la figure 1.

### DESCRIPTION DETAILLEE DE L'INVENTION

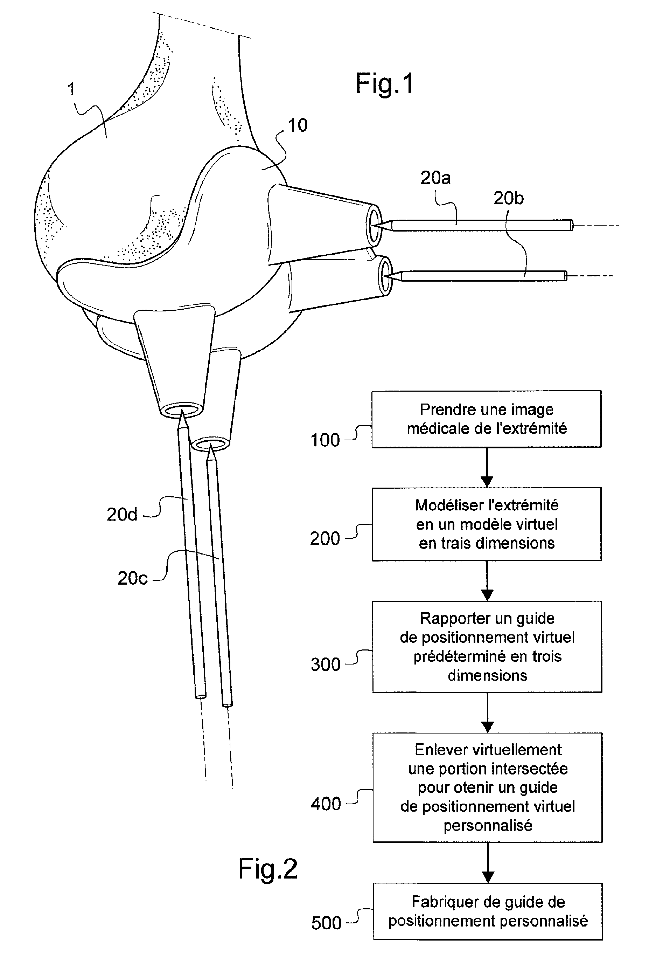

En référence aux figures 1 et 2, le procédé de fabrication selon l'invention est ici mis en oeuvre pour fabriquer un guide de positionnement 10 personnalisé d'ici au moins une broche sur l'extrémité distale 1 d'un fémur. Comme illustré à la figure 1, le guide de positionnement 10 personnalisé est ici destiné à positionner en service quatre broches 20a, 20b, 20c et 20d sur l'extrémité distale 1 du fémur.

Selon une première étape 100, au moins une image médicale est prise de l'extrémité distale 1 du fémur. Par exemple, l'image médicale est issue d'une Imagerie par Résonnance Magnétique (IRM).

Dans une deuxième étape 200, de façon connue en soi, à partir de l'image médicale, l'extrémité distale 1 du fémur est modélisée en un modèle virtuel en trois dimensions.

Puis, selon une troisième étape 300, un guide de positionnement virtuel prédéterminé en trois dimensions est rapporté sur le modèle virtuel de l'extrémité distale 1 du fémur.

Selon un mode de réalisation privilégié, le procédé selon l'invention comporte l'étape de choisir le guide de positionnement virtuel prédéterminé dans une liste de guides de positionnement virtuel qui sont chacun adaptés à une catégorie d'individus présente dans un échantillon de population. Les catégories sont par exemple caractérisées par l'un des paramètres suivants ou par une combinaison desdits paramètres : sexe d'un individu, taille d'un individu, poids d'un individu ...

Le guide de positionnement virtuel prédéterminé à rapporter est ainsi choisi dans la liste à partir des paramètres propres au patient, lesdits paramètres permettant de rattacher le patient à l'une desdites catégories. De préférence, le choix du guide de positionnement virtuel est automatique.

De façon avantageuse, le guide de positionnement virtuel prédéterminé est alors déjà sensiblement adapté à l'anatomie de l'extrémité distale 1 du fémur.

Dans une quatrième étape 400, une fois le guide de positionnement virtuel prédéterminé rapporté sur le modèle virtuel de l'extrémité 1, une portion intersectée du guide de positionnement virtuel prédéterminé par le modèle virtuel de l'extrémité 1 est déterminée. Puis, ladite portion intersectée est enlevée virtuellement du guide de positionnement virtuel prédéterminé de façon à obtenir un guide de positionnement virtuel personnalisé. La quatrième étape 400 permet ainsi d'affiner l'adaptation du guide de positionnement virtuel prédéterminé à l'anatomie de l'extrémité distale 1 du fémur.

Selon une dernière étape 500, et de façon connue en soi, le guide de positionnement 10 personnalisé est alors fabriqué à partir du guide de positionnement virtuel personnalisé par une opération de frittage de poudre.

Bien entendu l'invention n'est pas limitée au mode de réalisation décrit et on peut y apporter des variantes de réalisation sans sortir du cadre de l'invention tel que défini par les revendications.

En particulier, bien qu'ici l'extrémité distale 1 du fémur du patient ait été modélisée en un modèle virtuel en trois dimensions à partir d'une image médicale issue d'une IRM, l'extrémité distale 1 pourra être modélisée à partir d'un nombre plus important d'images et/ou à partir d'images médicales issues d'autres examens médicaux comme une radiographie, un scanner ou un arthroscanner.

Le procédé de fabrication de l'invention permet de fabriquer d'autres types de guide de positionnement personnalisé que celui illustré. Le guide de positionnement virtuel prédéterminé sera par exemple choisi en fonction du type de guide de positionnement personnalisé recherché. Par exemple, le procédé selon l'invention peut également être mis en oeuvre pour permettre la fabrication d'un guide de positionnement personnalisé de broches pour l'extrémité proximale d'un tibia. En outre, bien qu'ici le guide de positionnement personnalisé permette en service de guider le positionnement de quatre broches, le procédé selon l'invention peut également être mis en oeuvre pour fabriquer un guide de positionnement personnalisé qui soit destiné à guider en service un nombre différent de broches, par exemple une unique broche.

Bien qu'ici le guide de positionnement virtuel prédéterminé soit choisi à partir de la liste et des paramètres propres au patient, le guide de positionnement virtuel prédéterminé pourra être choisi autrement. Par exemple, à partir de l'image médicale, une ou plusieurs mesures peuvent être relevées (ratio Antéro-Postérieur/Médio-Latéral...). Le guide de positionnement virtuel prédéterminé est alors choisi en fonction de cette ou de ces mesures.

En outre, le choix du guide de positionnement virtuel prédéterminé peut être fait, non pas automatiquement, mais par un chirurgien.

Le procédé selon l'invention pourra comporter d'autres d'étapes que celles indiquées. Par exemple, entre la quatrième étape 400 d'enlèvement de la portion intersectée et l'étape 500 de fabrication, le procédé selon l'invention pourra comporter une étape où le guide de positionnement virtuel personnalisé est retouché, de façon automatique ou par le chirurgien, afin de l'adapter encore davantage à l'anatomie du patient. Pour cette étape, il sera tenu compte par exemple du ratio Antéro-Postérieur/ Médio-Latéral, d'une étude de la position relative du fémur par rapport au tibia tout au long d'un mouvement de flexion du genou associé ...

Bien qu'ici le guide de positionnement soit destiné à positionner au moins une broche, le guide de positionnement pourra être destiné à positionner d'autres organes de positionnement comme par exemple des clous, des vis, des tiges filetées ... Par organe de positionnement, on entendra tout moyen permettant le maintien d'un guide ou similaire sur un os.

## Revendications

1. Procédé de fabrication d'un guide de positionnement (10) personnalisé d'au moins un organe de positionnement sur une extrémité distale (1) d'un fémur ou sur une extrémité proximale d'un tibia d'un patient, le procédé comportant l'étape (200) de modéliser l'extrémité souhaitée en un modèle virtuel en trois dimensions à partir d'au moins une image médicale de ladite extrémité, le procédé étant **caractérisé en ce qu**'il comporte les étapes de :
- rapporter (300) sur le modèle virtuel de l'extrémité un guide de positionnement virtuel prédéterminé en trois dimensions ;
- enlever (400) virtuellement du guide de positionnement virtuel prédéterminé une portion intersectée par le modèle virtuel de l'extrémité pour obtenir un guide de positionnement virtuel personnalisé;
- fabriquer (500) le guide de positionnement personnalisée par frittage de poudre à partir du guide de positionnement virtuel personnalisé.

2. Procédé de fabrication selon la revendication 1, comportant l'étape de choisir le guide de positionnement virtuel prédéterminé dans une liste de guides de positionnement virtuel qui sont chacun adaptés à une catégorie d'individus présente dans un échantillon de population.

3. Procédé de fabrication selon la revendication 1, dans lequel l'image médicale utilisée pour modéliser l'extrémité est issue d'une Imagerie par Résonnance Magnétique.
